(19) **Europäisches Patentamt European Patent Office Office européen des brevets**

(11) **EP 2 008 998 B1**

(12) **EUROPEAN PATENT SPECIFICATION**

(45) Date of publication and mention of the grant of the patent:
**05.06.2013 Bulletin 2013/23**

(21) Application number: **07737056.7**

(22) Date of filing: **12.04.2007**

(51) Int Cl.:
*C07C 319/14* (2006.01)    *C07C 335/32* (2006.01)
*C08G 75/04* (2006.01)    *G02B 1/04* (2006.01)
*C08G 18/38* (2006.01)

(86) International application number:
**PCT/JP2007/000399**

(87) International publication number:
**WO 2007/129450 (15.11.2007 Gazette 2007/46)**

(54) **PROCESS FOR PRODUCTION OF (POLY)THIOL COMPOUND FOR USE AS OPTICAL MATERIAL**

VERFAHREN ZUR HERSTELLUNG EINER (POLY)THIOLVERBINDUNG ZUR VERWENDUNG ALS OPTISCHES MATERIAL

PROCÉDÉ DE PRODUCTION D'UN COMPOSÉ DE (POLY)THIOL UTILISÉ COMME MATÉRIAU OPTIQUE

(84) Designated Contracting States:
**DE FR GB IT**

(30) Priority: **19.04.2006 JP 2006115289**

(43) Date of publication of application:
**31.12.2008 Bulletin 2009/01**

(73) Proprietor: **Mitsui Chemicals, Inc.**
**Tokyo 105-7117 (JP)**

(72) Inventors:
• **KUMA, Shigetoshi**
**Omuta-shi**
**Fukuoka 836-8610 (JP)**
• **SAKATA, Michiharu**
**Omuta-shi**
**Fukuoka 836-8610 (JP)**
• **KOBAYASHI, Seiichi**
**Omuta-shi**
**Fukuoka 836-8610 (JP)**

(74) Representative: **Wills, Andrew Jonathan et al**
**Mewburn Ellis LLP**
**33 Gutter Lane**
**London**
**EC2V 8AS (GB)**

(56) References cited:
**JP-A- 02 270 859      JP-A- 07 252 207**
**JP-A- 2006 284 920**

Remarks:
The file contains technical information submitted after the application was filed and not included in this specification

EP 2 008 998 B1

**Description**

TECHNICAL FIELD

The present invention relates to a (poly)thiol compound fo an optical material and a process for producing the same, and a polymerizable composition composed of the (poly) thiol compound and a polyiso(thio)cyanate compound, which is used as an optical material of a polyurethane based lens exhibiting excellent optical properties.

BACKGROUND ART

As a method for the production of a thiol compound, many methods have been known from the past. As the method, there can be exemplified, for example, a method including reducing a disulfide compound, a method including reacting an organic halide with an alkali metal hydrosulfide salt or an alkali metal sulfide salt such as sodium hydrosulfide, potassium hydrosulfide or the like, a method including reacting an organic halide or alcohol with thiourea to produce an isothiuronium salt and hydrolyzing the isothiuronium salt with a base, a method including producing a Bunte salt, a method including producing dithiocarbamic acid ester, a method including using a Grignard reagent and sulfur, a method including fragmentizing a C-S bond of sulfide, a method including ring-opening episulfide, a method including reacting a compound having a carbonyl group as a starting material with hydrogen sulfide, a method including adding hydrogen sulfide or thioacetic acid to alkene.

Of the methods, the method for the production of a thiol compound by producing an isothiuronium salt from an organic halide or alcohol brings a high yield, produces a small amount of by-product, is excellent in operability, results in obtaining a product with good quality in many cases as compared to other production methods. Therefore, this method is one of methods for the production of a thiol compound which is generally used as the best method,

Furthermore, for the reaction of an organic (poly)halogen compound or a (poly)alcohol compound with thiourea, it is known that a method for the production of a (poly) thiol compound by adding sulfuric acid to produce an isothiuronium salt is capable of effectively producing a (poly)thiol compound in a high yield and at low cost (refer to Patent Document 1).

At that time, thiourea in use is produced from lime nitrogen and hydrogen sulfide, or calcium hydrosulfide. Further, it is known that a thiourea-containing solution is purified by a strong basic ion exchange resin (refer to Patent Document 2).

Furthermore, there has been described that a poly(thio)urethane resin obtained by reacting a (poly) thiol compound obtained by this production method with a polyiso(thio)cyanate compound is colorless and transparent, has a high refractive index and a low dispersion, is excellent in impact resistance, dyeing property, processability, and is one of resins which are optimum for plastic lenses of optical materials (refer to Patent Documents 3, 4, 5 and EP 0378895).

However, the (poly)thiol compound obtained even in the above production method caused a problem of coloring in many times and was difficult to be stably produced.

Patent Document 1: Japanese Patent Laid-open No. 2001-39944
Patent Document 2: Japanese Patent Laid-open No. S48(1973)-49722
Patent Document 3: Japanese Patent Laid-open No. H9(1997)-110955
Patent Document 4: Japanese Patent Laid-open No. H9(1997)-110956
Patent Document 5: Japanese Patent Laid-open No. H7(1995)-252207

DISCLOSURE OF THE INVENTION

However, a (poly)thiol compound produced by the conventional methods including producing an isothiuronium salt caused a problem of coloring, or a poly(thio)urethane resin obtained by using the (poly)thiol compound caused a problem of coloring or whitening in some cases.

For that reason, there has been demanded that the occurrence of such problems in the methods including producing an isothiuronium salt should be suppressed to the utmost, and an industrial method for the production of a (poly)thiol compound without causing coloring should be developed. Accordingly, a plastic lens composed of a poly(thio) urethane resin without causing coloring or whitening needed to be provided to the world in a stable manner.

The present invention relates to a process for producing a (poly) thiol compound by reacting an organic (poly) halogen compound or a (poly)alcohol compound with thiourea to produce an isothiuronium salt and hydrolyzing the obtained isothiuronium salt, and a process for producing a colorless and transparent (poly) thiol compound in which coloring is suppressed. Furthermore, the invention is to provide, by polymerizing the (poly)thiol compound obtained by the process of the present invention with a polyiso(thio)cyanate compound, a colorless and transparent poly(thio)urethane resin in which coloring or whitening is suppressed, and a plastic lens which is useful as an optical material.

In order to solve the above objects, the present inventors have conducted an extensive study and as a result, have confirmed that coloring of a poly (thio) urethane resin is caused by the color tone of a (poly)thiol compound in use.

Furthermore, to search for the cause of coloring of a (poly)thiol compound, the inventors have conducted an extensive study on a process for producing a (poly)thiol compound prepared by producing an isothiuronium salt from an organic (poly) halogen compound or a (poly) alcohol compound and hydrolyzing the isothiuronium salt, and the production conditions. As a result, the inventors have found conditions for the production of a colorless and transparent (poly) thiol compound in which coloring is suppressed. However, even if the production conditions were the same, the (poly)thiol compound was colored in some cases and was difficult to be stably produced.

Even though the production conditions were the same, to solve the aforementioned problem of coloring of the (poly) thiol compound, they have checked in detail the quality of thiourea used for producing an isothiuronium salt. They have continued an extensive study on how the purity of thiourea and the quality of a trace of impurities contained in thiourea have influence on coloring of the obtained (poly)thiol, and whitening or coloring of the poly(thio)urethane resin. As a result, surprisingly, when the amount of impurities contained in thiourea is not less than a specific amount, coloring of the obtained (poly) thiol compound has been clearly observed. They have conducted an extensive study on the specification of the impurities and as a result, have specified that a main ingredient of the impurities is calcium. As a result, they have found that, when a (poly) thiol compound is produced with thiourea having a calcium content of not more than a specific amount as a starting material, a colorless and transparent (poly)thiol compound in which coloring is suppressed can be stably obtained. Furthermore, they have found that a colorless and transparent poly(thio)urethane resin in which coloring and whitening are suppressed by using the compound is obtained. Thus, the present invention has been completed.

That is, the present invention is specified by the following matters:

(1) a process for producing a (poly) thiol compound for an optical material comprising:

reacting an organic (poly)halogen compound or a (poly)alcohol compound with thiourea to produce an isothiuronium salt, and

hydrolyzing the obtained isothiuronium salt in the presence of aqueous ammonia to produce a (poly)thiol compound,

in which the calcium content in the thiourea is not more than 1.0 wt %;

(2) the process for producing a (poly)thiol compound for an optical material as set forth in (1) above, in which the (poly) thiol compound has a sulfur atom in addition to a thiol group;

(3) the process for producing a (poly)thiol compound for an optical material as set forth in (2) above, in which the (poly) thiol compound having a sulfur atom in addition to a thiol group has one or two or more kinds selected from the group consisting of 1,2-bis[(2-mercaptoethyl)thio]-3-mercaptopropane, 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane as main ingredients;

(4) a process of producing a resin comprising producing a (poly)thiol compound by the process as set forth in any one of (1) to (3) above, mixing it with a polyiso (thio) cyanate compound; and curing the resulting polymerizable composition to produce the resin;

(5) the process of (4) used to produce an optical material containing the resin; and

(6) the process of (4) used to produce a lens containing the resin.

In the above (5) and (6), the phrase "containing the resin" refers to both a case in which the entire optical material or the entire lens is composed of the resin and a case in which a part of the optical material or the lens is composed of the resin.

The process for producing a (poly)thiol compound fo an optical material of the present invention is suitable for an industrial application, and is capable of stably obtaining a colorless and transparent (poly)thiol compound in which coloring is suppressed. The poly(thio)urethane resin obtained by using a polymerizable composition composed of a (poly)thiol compound for an optical material obtained in accordance with the production process of the present invention and a polyiso (thio) cyanate compound is colorless and transparent, in which coloring and whitening are suppressed. According to the present invention, it is possible to provide a colorless and transparent polyurethane based lens useful as an optical material and a transparent material in a stable manner which contribute to the development of the related fields.

BEST MODE FOR CARRYING OUT THE INVENTION

The present invention will be illustrated in detail below. The present invention relates to a process for producing a (poly) thiol compound for an optical material by reacting an organic (poly)halogen compound or a (poly)alcohol compound with thiourea to produce an isothiuronium salt and hydrolyzing the obtained isothiuronium salt. The content of calcium in thiourea used for the present invention is not more than a specific amount. That is, thiourea having a calcium content of not more than 1.0 wt % is used.

Thiourea to be used as a starting material for forming an isothiuronium salt is mainly produced by reacting lime nitrogen with hydrogen sulfide. Examples of impurities contained in thiourea include unreacted lime nitrogen, and further by-produced calcium hydroxide. That is, when calcium is contained in thiourea in excess of a specific amount, the obtained (poly)thiol compound is colored, and a polymerizable composition obtained by mixing with a polyiso (thio) cyanate compound and the obtained resin are colored or whitened.

The calcium content in thiourea used for the present invention is preferably from 0.0005 to 1.0 wt %, more preferably from 0.0005 to 0.5 wt % both inclusive, and further preferably from 0.0005 to 0.2 wt % both inclusive from the viewpoint of suppression of coloring and whitening.

When the calcium content is not more than 1.0 wt %, a (poly) thiol compound produced by using the thiourea is colorless and transparent, in which coloring is suppressed. Further, the poly(thio)urethane resin obtained by polymerizing the produced (poly)thiol compound with polyiso(thio)cyanate composes a colorless and transparent poly(thio)urethane based lens in which whitening and coloring are suppressed.

The calcium content is measured in the following manner. Thiourea is made into an aqueous solution, and then its calcium content is quantitatively analyzed by an ion chromatographic method.

The calcium content can be reduced by employing a method such as purification, acid treatment, or recrystallization, and must not be more than 1.0 wt %. Specifically, the calcium content can be reduced, for example, by acid treatment, e.g. using hydrochloric acid or sulfuric acid. It can also be reduced by a recrystallization method using an aqueous system.

The organic (poly)halogen compound as the other starting material is a compound having one or more halogen atoms in a molecule, and is not particularly restricted in terms of quality.

Concrete examples of the starting material organic (poly)halogen compound include bis(2,3-dichloropropyl)sulfide, 1,1,1-tris(chloromethyl)propane, 1,1,1-tris(bromomethyl)propane, 1,2-bis(2-chloroethylthio)-3-chloropropane, 1,2-bis(2-bromoethylthio)-3-bromopropane, 1,3-bis(2-chloroethylthio)-2-chloropropane, 1,3-bis(2-bromoethylthio)-2-bromopropane, 2,5-bis(chloromethyl)-1,4-dithiane, 2,5-bis(bromomethyl)-1,4-dithiane, 4,8-dichloromethyl-1,11-dichloro-3,6,9-trithiaundecane, 4,8-dichloromethyl-1,11-dichloro-3,6,9-trithiaundecane, 5,7-dichloromethyl-1,11-dichloro-3,6,9-trithiaundecane, 4,8-dibromomethyl-1,11-dibromo-3,6,9-trithiaundecane, 4,7-dibromomethyl-1,11-dibromo-3,6,9-trithiaundecane, 5,7-dibromomethyl-1,11-dibromo-3,6,9-trithiaundecane, 1,5,9,13-tetrachloro-3,7,11-trithiatridecane, 1,5,9,13-tetrabromo-3,7,11-trithiatridecane, 1,2,6,7-tetrachloro-4-thiaheptane and 1,2,6,7-tetrabromo-4-thiaheptane, but the present invention is not restricted to these exemplified compounds.

The (poly)alcohol compound as the other starting material is a compound having one or more hydroxy groups in a molecule, and is not particularly restricted in terms of quality. Concrete examples thereof include bis(2,3-dihydroxy)sulfide, 1,1,1-tris(hydroxymethyl)propane, 1,2-bis(2-hydroxyethylthio)-3-hydroxypropane, 1,3-bis(2-hydroxyethylthio)-2-hydroxypropane, 2,5-bis(hydroxymethyl)-1,4-dithiane,
4,8-dihydroxymethyl-1,11-dihydroxy-3,6,9-trithiaundecane, 4,7-dihydroxymethyl-1,11-dihydroxy-3,6,9-trithiaundecane, 5,7-dihydroxymethyl-1,11-dihydroxy-3,6,9-trithiaundecane, 1,5,9,13-tetrahydroxy-3,7,11-trithiatridecane, 1,2,6,7-tetrahydroxy-4-thiaheptane and pentaerythritol,
but the present invention is not restricted to these exemplified compounds.

In the present invention, a process including reacting an organic (poly)halogen compound or a (poly)alcohol compound with thiourea is preferably carried out in a solvent. The solvent used at that time is, for example, water, alcohol other than a starting material or an organic halogen compound.

As alcohol, for example, methanol, ethanol, isopropanol, butanol, or methoxyethanol are preferably used.

Examples of the organic halogen compound include dichloromethane, dichloroethane, chloroform, chlorobenzene, o-dichlorobenzene and p-dichlorobenzene.

Hydrolysis which subsequently carried out after producing an isothiuronium salt is conducted by using aqueous ammonia. The use of ammonia water gives remarkable good results.

The amount of the base used is generally in the range of 1.0 to 3.0 equivalents both inclusive for obtaining the preferable results, and in the range of 1.0 to 2.0 equivalents both inclusive for obtaining the further preferable results, based on the number of halogen atoms bonded to the organic halogen compound or the amount of hydrohalogenated acid which is well used in case of (poly)alcohols.

The reaction temperature at the time of hydrolysis is generally in the range of 0 to 100 degree centigrade, and preferably in the range of 20 to 70 degree centigrade.

As the solvent used for hydrolysis, there are preferably used, for example, water; alcohols such as methanol, ethanol, isopropanol, butanol and methoxyethanol; aromatic hydrocarbon solvents such as toluene and xylene; and halogen solvents such as chlorobenzene and dichlorobenzene.

The reaction for producing an isothiuronium salt in the prior step is carried out in a water solvent and the isothiuronium salt may be subjected to hydrolysis as it is without taking out a reactant. In that case, an aromatic hydrocarbon solvent such as toluene or xylene is added to the reaction system for carrying out hydrolysis in a double-layer system. In such a process, the generated (poly)thiol compound is extracted to an organic solvent, whereby washing procedures carried out thereafter are conducted effectively and within a short period of time in some cases; therefore, it is preferable.

The thus-obtained reaction solution containing such a (poly)thiol compound in the present invention is usually subjected, if necessary, to various washing treatments including acid washing, base washing and water washing for removing the solvent and then filtering to obtain as a product. Furthermore, the solution may be purified by various purification methods such as distillation, column chromatography, or recrystallization.

According to the production process of the present invention, a colorless and transparent (poly) thiol compound in which coloring is suppressed is obtained. The (poly)thiol compound obtained in the present invention may have a sulfur atom in addition to a thiol group. Specifically, for example, in the following compounds, an effect of the present invention is more remarkably obtained.

Examples thereof include (poly)thiol compounds having main ingredients of one or two or more kinds selected from the group consisting of 1,2-bis[(2-mercaptoethyl)thio]-3-mercaptopropane, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, 2,5-dimercaptomethyl-1,4-dithiane, 1,1,3,3-tetramercaptomethyl-2-thiapropane, bis(2,3-dimercaptopropyl)sulfide, 1,1,1-tris(mercaptomethyl)propane, 1,5,9,13-tetramercapto-3,7,11-trithiatridecane and tetramercaptomethylmethane, but the present invention is not restricted to these exemplified compounds.

The polyiso (thio) cyanate compound used in the present invention is a compound having at least two or more iso(thio)cyanate groups in a molecule, and is not particularly limited. Concrete examples thereof include aliphatic polyisocyanate compounds such as hexamethylene diisocyanate, 2,2-dimethylpentane diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecane triisocyanate, 1,3,6-hexamethylene triisocyanate, 1,8-diisocyanato-4-isocyanatomethyloctane, bis(isocyanatoethyl)carbonate, bis(isocyanatoethyl)ether, lysine diisocyanatomethyl ester and lysine triisocyanate;

polyisocyanate compounds having an aromatic compound such as xylylene diisocyanate, 1,2-diisocyanatobenzene, 1,3-diisocyanatobenzene, 1,4-diisocyanatobenzene, 2,4-diisocyanatotoluene, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, biphenyl diisocyanate, toluidine diisocyanate, 4,4'-methylenebis(phenyl isocyanate), 4,4'-methylenebis(2-methylphenyl isocyanate), bibenzyl-4,4'-diisocyanate, bis(isocyanatophenyl)ethylene, bis(isocyanatoethyl)benzene, bis(isocyanatopropyl)benzene, $\alpha,\alpha,\alpha'$,$\alpha'$-tetramethylxylylene diisocy-anate, bis(isocyanatobutyl)benzene, bis(isocyanatomethyl)naphthalene, bis(isocyanatomethylphenyl)ether, bis(isocyanatoethyl)phthalate and 2,6-di(isocyanatomethyl)furan;

sulfur-containing aliphatic polyisocyanate compounds such as bis(isocyanatomethyl)sulfide, bis(isocyanatoethyl)sulfide, bis(isocyanatopropyl)sulfide, bis(isocyanatohexyl)sulfide, bis(isocyanatomethyl)sulfone, bis(isocyanatomethyl)disulfide, bis(isocyanatoethyl)disulfide, bis(isocyanatopropyl)disulfide, bis(isocyanatomethylthio)methane, bis(isocyanatoethylthio)methane, bis(isocyanatomethylthio)ethane, bis(isocyanatoethylthio)ethane, 1,5-diisocyanato-2-isocyanatomethyl-3-thiapentane, 1,2,3-tris(isocyanatomethylthio)propane, 1,2,3-tris(isocyanatoethylthio)propane, 3,5-dithia-1,2,6,7-heptane tetraisocyanate, 2,6-diisocyanatomethyl-3,5-dithia-1,7-heptane diisocyanate, 2,5-diisocyanate methyl thiophene and 4-isocyanatoethylthio-2,6-dithia-1,8-octane diisocyanate;

aromatic sulfide based polyisocyanate compounds such as 2-isocyanatophenyl-4-isocyanatophenyl sulfide, bis(4-isocyanatophenyl)sulfide and bis(4-isocyanatomethylphenyl)sulfide;

aromatic disulfide based polyisocyanate compounds such as bis(4-isocyanatophenyl)disulfide, bis(2-methyl-5-isocyanatophenyl)disulfide, bis(3-methyl-5-isocyanatophenyl)disulfide, bis(3-methyl-6-isocyanatophenyl)disulfide, bis(4-methyl-5-isocyanatophenyl)disulfide and bis(4-methoxy-3-isocyanatophenyl)disulfide;

sulfur-containing alicyclic polyisocyanate compounds such as 2,5-diisocyanatotetrahydrothiophene, 2,5-diisocyanatomethyltetrahydrothiophene, 3,4-diisocyanatomethyltetrahydrothiophene, 2,5-diisocyanato-1,4-dithiane, 2,5-diisocyanatomethyl-1,4-dithiane, 4,5-diisocyanato-1,3-dithiolane, 4,5-bis(isocyanatomethyl)-1,3-dithiolane and 4,5-diisocyanatomethyl-2-methyl-1,3-dithiolane;

aliphatic polyisothiocyanate compounds such as 1,2-diisothiocyanatoethane and 1,6-diisothiocyanatohexane;

alicyclic polyisothiocyanate compounds such as cyclohexane diisothiocyanate;

aromatic polyisothiocyanate compounds such as 1,2-diisothiocyanatobenzene, 1,3-diisothiocyanatobenzene, 1,4-diisothiocyanatobenzene, 2,4-diisothiocyanatotoluene, 2,5-diisothiocyanato-m-xylene, 4,4'-methylenebis(phenyl isothiocyanate), 4,4'-methylenebis(2-methylphenyl isothiocyanate), 4,4'-methylenebis(3-methylphenyl isothiocyanate), 4,4'-diisothiocyanatobenzophenone, 4,4'-diisothiocyanato-3,3'-dimethylbenzophenone and bis(4-isothiocyanatophenyl) ether;

further, carbonyl polyisothiocyanate compounds such as 1,3-benzenedicarbonyl diisothiocyanate, 1,4-benzenedicarbonyl diisothiocyanate and (2,2-pyridine)-4,4-dicarbonyl diisothiocyanate; sulfur-containing aliphatic polyisothiocyanate compounds such as thiobis(3-isothiocyanatopropane), thiobis(2-isothiocyanatoethane) and dithiobis(2-isothiocyanatoethane);

sulfur-containing aromatic polyisothiocyanate compounds such as 1-isothiocyanato-4-[(2-isothiocyanato)sulfonyl]benzene, thiobis(4-isothiocyanatobenzene), sulfonyl(4-isothiocyanatobenzene) and dithiobis(4-isothiocyanatobenzene);

sulfur-containing alicyclic polyisothiocyanate compounds such as 2,5-diisothiocyanatothiophene and 2,5-diisothiocyanato-1,4-dithiane; and

compounds having an isocyanato group and an isothiocyanate group such as 1-isocyanato-6-isothiocyanatohexane, 1-isocyanato-4-isothiocyanatocyclohexane, 1-isocyanato-4-isothiocyanatobenzene, 4-methyl-3-isocyanato-1-isothiocyanatobenzene, 2-isocyanato-4,6-diisothiocyanato-1,3,5-triazine, 4-isocyanatophenyl-4-isothiocyanatophenyl sulfide and 2-isocyanatoethyl-2-isothiocyanatoethyl disulfide.

Furthermore, there can be used their halogen substituted compounds such as chlorine substituted compounds or bromine substituted compounds; their alkyl substituted compounds, their alkoxy substituted compounds; their nitro substituted compounds; prepolymer type modified compounds modified with polyhydric alcohols; carbodiimide-modified compounds; urea-modified compounds; biuret-modified compounds; and dimerization or trimerization reaction compounds. These compounds may be used singly, or two or more compounds may be used in combination.

The proportion of the (poly)thiol compound and the polyiso (thio) cyanate compound is not particularly limited, but the molar ratio is usually in the range of 0.3 to 2.0 both inclusive (SH group/NCO group), preferably in the range of 0.7 to 2.0 both inclusive, and further preferably in the range of 0.7 to 1.3 both inclusive. When the proportion is within the above range, it is possible to satisfy each performance criterion such as refractive index or heat resistance which is desired for an optical material and to provide a transparent material of a plastic lens with a good balance.

For purposes of improvement of general properties, operability and polymerization reactivity of the polyurethane based resign, other substances may be added, in addition to the ester compound and iso(thio)cyanate compound forming the urethane resin. For example, in addition to a urethane-forming starting material, one or two or more active hydrogen compounds such as amines, epoxy compounds, olefin compounds, carbonate compounds, ester compounds, metals, metal oxides, organic metal compounds, or inorganic substances may be added.

Further, a variety of substances such as a chain extender, a crosslinking agent, a photostabilizer, a UV absorber, an antioxidant, an oil soluble dye, a filler, a releasing agent, and a blueing agent, may be added, depending on the purposes, as in a known molding method. In order to adjust to a desired reaction rate, a thiocarbamic acid S-alkyl ester or a known reaction catalyst used for producing polyurethane may be added as appropriate. The lens formed of the polyurethane resin can be usually obtained by casting polymerization.

Specifically, the (poly)thiol compound obtained by the production process of the present invention is mixed with a polyiso(thio)cyanate compound to obtain a mixed solution containing the polymerizable composition. This mixed solution is degassed according to a proper method as needed, and then injected into a mold and usually slowly heated from a low temperature to a high temperature for polymerization.

The thus-obtained polyurethane based resin has a high refractive index, a low dispersion, excellent heat resistance and durability, light weight, and excellent impact resistance and the occurrence of whitening is further suppressed. Thus it is suitable as an optical material and a transparent material for a spectacle lens or a camera lens, for example.

Furthermore, the lens which is obtained by using the polyurethane resin may be, if necessary, subjected to physical or chemical treatment such as surface abrasion treatment, antistatic treatment, hard coat treatment, non-reflective coat treatment, dyeing treatment and polarizing treatment, for prevention of reflection, enhancement of hardness, improvement of abrasion resistance, improvement of chemical resistance, supply of anticlouding, or supply of fashionability.

EXAMPLES

The present invention is now illustrated in detail below with reference to Examples. Thiourea and the obtained (poly)thiol compound and the polyurethane based resin obtained by polymerization were analyzed in the following manner.

- Content of calcium in thiourea: Thiourea was dissolved in water to give an aqueous solution, and then the calcium content was measured by an ion chromatographic method.
- Color of polythiol (APHA: American Public Healthy Association) : APHA was employed as an analyzing item for evaluating the color of the obtained (poly) thiol compound. APHA was measured in accordance with JIS K 0071-1. Specifically, APHA was obtained by comparing the color of a sample to diluted standard solution having an equivalent concentration using a standard solution prepared by melting a reagent of platinum and cobalt. Its degree was taken as a measurement value. The smaller the value was, the better the color was.
- Color of polythiol (Y.I): Yellow index (Y.I.) was employed as an analyzing item for evaluating the color more in detail.

Y.I. was measured by using a colorimeter CT-210 (a product of Minolta Co., Ltd.). Firstly, distilled water was fed into a cell CT-A20 having an optical path length of 20 mm, and a white calibration was performed as Y=100.00, x=0.3101 and y=0.3162. Thereafter, a sample was fed into the same cell and the color measurement was carried out. The measurement results, x and y values, were used to calculate Y.I. according to the following formula:

$$Y.I. = (234 \times x + 106 \times y + 106)/y \qquad (1)$$

This Y.I value was taken as a numerical value of the color of polythiol. The higher the numerical value was, the greater the coloring degree was.

When a liquid polythiol was measured, it was fed into a cell having a thickness of 10 mm for the measurement.

• Color of polyurethane based resin (Y.I): A colorimeter, CT-210, manufactured by Minolta Co., Ltd. was used to measure the Y. I. of a plastic lens obtained from the polyurethane resin. A round flat plate with a thickness of 9 mm and $\phi$ of 75 mm was produced by cast polymerization, and then measured on the color coordination, x and y. Based on the resulting x and y values, the above equation (1) was used to determine the yellow index (Y. I).

• Loss degree of transparency: As an analyzing item for evaluating the transparency of the plastic lens containing a polyurethane based resin, the loss degree of transparency was employed. The loss degree of transparency was obtained in the following means. The lens plate of a circular flat plate having a thickness of 9 mm and $\phi$75 mm was prepared. Then, the lens plate was irradiated with a light source (Luminar Ace LA-150A, a product of Hayashi Watch Works Co., Ltd.) for measuring the loss degree of transparency with a gray scale image processing unit. Captured images were expressed in numbers by gray scale image processing to obtain the loss degree of transparency. When the loss degree of transparency is not more than 30, it was indicated with o, while, when it was greater than 30, it was indicated with $\times$.

Reduction of calcium content in thiourea

The calcium (Ca) content in thiourea was reduced by the following procedure.

Into a 2-liter, 4-necked flask equipped with a stirrer, a reflux condensing water separator, a nitrogen gas purge tube and a thermometer were introduced 1,530 weight parts of distilled water and 470.0 weight parts of thiourea with the purity of 98.2% containing Ca of 1.5 wt %. The resulting material was heated to 40 degree centigrade for removing an insoluble matter by filtering. Thereafter, the filtrate was cooled down to 5 degree centigrade, and thiourea was precipitated and crystallized at the same temperature for 3 hours. Thiourea was taken out by filtering, and vacuum-dried at 40 degree centigrade under 700 Pa to obtain 368.6 g of thiourea having a Ca content of 0.07 wt %.

Furthermore, in other Examples and Comparative Examples, the crystallization time was properly adjusted by using the aforementioned method to obtain various thioureas having different Ca contents.

Example 1

Synthesis of (poly)thiol compound having 1,2-bis[(2-mercaptoethyl)thio]-3-mercaptopropane as main ingredient

Into a 2-liter, 4-necked flask equipped with a stirrer, a reflux condensing water separator, a nitrogen gas purge tube and a thermometer were introduced 169 weight parts (2.16 mol) of 2-mercaptoethanol and 76.0 weight parts of water. At 30 degree centigrade, 91.9 weight parts (1.08 mol) of 47 wt % aqueous sodium hydroxide solution was added dropwise thereto over 30 minutes, and then 99.9 weight parts (1.08 mol) of epichlorohydrin was added dropwise at the same temperature over 3 hours, and the resulting solution was matured for 1 hour. Next, 450.0 weight parts (4.32 mol) of 35 wt % hydrochloric acid water and 246.9 weight parts (3.24 mol) of thiourea with the purity of 99.90% having a calcium content of 0.05 wt % obtained by recrystallization in advance were introduced, and the resulting solution was matured under reflux at 110 degree centigrade for 3 hours for producing a thiuronium salt. The solution was cooled down to 60 degree centigrade, and then 450.0 weight parts of toluene and 331.1 weight parts (4.86 mol) of 25 wt % aqueous ammonia solution were introduced thereinto for carrying out hydrolysis to obtain a toluene solution of polythiol having 1,2-bis[(2-mercaptoethyl)thio]-3-mercaptopropane as a main ingredient. The toluene solution was subjected to acid washing and water washing for removing toluene and a trace of water under heat and reduced pressure. Thereafter, 268.7 weight parts of polythiol having 1,2-bis[(2-mercaptoethyl)thio]-3-mercaptopropane as a main ingredient was obtained by filtering. APHA of the obtained polythiol was 10, while Y.I thereof was 0.70.

Production of plastic lens

52 weight parts of m-xylylene diisocyanate, 0.015 weight parts of dibutyltin dichloride as a curing catalyst, 0.10 weight part of Zelec UN (product name, acid phosphoric acid alkyl ester, a product of Stepan Co.) as an internal mold releasing agent and 0.05 weight parts of Viosorb 583 (product name, a product of Kyodo Chemical Co., Ltd.) as an ultraviolet absorber were mixed and dissolved at 20 degree centigrade. After mixing and dissolving were confirmed, subsequently into this mixed and dissolved solution was introduced 48 weight parts of polythiol having 1,2-bis[(2-mercaptoethyl)thio]-3-mercaptopropane as a main ingredient obtained as in the above, and the resulting solution was mixed to give a uniform mixed solution. This uniform solution was degassed at 600 Pa for 1 hour. Thereafter, the resulting solution was filtered using a 3-$\mu$m Teflon (registered trademark) filter, and then injected into a mold equipped with a glass mold and tapes. This mold was put into an oven and then gradually heated from 10 to 120 degree centigrade at which polymerization was conducted for 18 hours. After completion of polymerization, the mold was taken out from the oven and a resin was released from the mold. The obtained resin was additionally annealed at 120 degree centigrade for 3 hours. Y.I. of the obtained resin was 4.5 and the loss degree of transparency was 20. So, the evaluation was indicated with "o" on the loss degree of transparency. The evaluation results are shown in Table 1.

Example 2

Polythiol having 1,2-bis[(2-mercaptoethyl)thio]-3-mercaptopropane as a main ingredient was synthesized in the same manner as in Example 1, except that thiourea with the purity of 99.70% having a calcium content of 0.20 wt % obtained by recrystallization in advance was used instead of thiourea used in Example 1. APHA of the obtained polythiol having 1,2-bis[(2-mercaptoethyl)thio]-3-mercaptopropane as a main ingredient was 10, while Y.I. thereof was 0.81. Using this polythiol, a plastic lens was produced and evaluated in the same manner as in Example 1. The evaluation results of the obtained plastic lens are shown in Table 1.

Example 3

Polythiol having 1,2-bis[(2-mercaptoethyl)thio]-3-mercaptopropane as a main ingredient was synthesized in the same manner as in Example 1, except that thiourea with the purity of 99.20% having a calcium content of 0.70 wt % obtained by recrystallization in advance was used instead of thiourea used in Example 1. APHA of the obtained polythiol having 1,2-bis[(2-mercaptoethyl)thio]-3-mercaptopropane as a main ingredient was 10, while Y.I. thereof was 0.93. Using this polythiol, a plastic lens was produced and evaluated in the same manner as in Example 1. The evaluation results of the obtained plastic lens are shown in Table 1.

Example 4

Polythiol having 1,2-bis[(2-mercaptoethyl)thio]-3-mercaptopropane as a main ingredient was synthesized in the same manner as in Example 1, except that thiourea with the purity of 99.00% having a calcium content of 0.90 wt % obtained by recrystallization in advance was used instead of thiourea used in Example 1. APHA of the obtained polythiol having 1,2-bis[(2-mercaptoethyl)thio]-3-mercaptopropane as a main ingredient was 10, while Y.I. thereof was 0.95. Using this polythiol, a plastic lens was produced and evaluated in the same manner as in Example 1. The evaluation results of the obtained plastic lens are shown in Table 1.

Example 5

Synthesis of polythiol having 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane as main ingredients
Into a 2-liter, 4-necked flask equipped with a stirrer, a reflux condensing water separator, a nitrogen gas purge tube and a thermometer were introduced 89.1 weight parts (1.14 mol) of 2-mercaptoethanol, 44.8 weight parts of water and 0.4 weight parts of 47 wt % aqueous sodium hydroxide solution. At 10 degree centigrade, 107.3 weight parts (1.16 mol) of epichlorohydrin was added dropwise over 4 hours, and the resulting solution was matured for 1 hour. Next, 261.6 weight parts (0.58 mol) of 16.9 wt % aqueous sodium sulfide solution was added dropwise thereto at 25 degree centigrade over 1 hour, and the resulting solution was matured at the same temperature for 3 hours. Subsequently, 211.8 weight parts (2.78 mol) of thiourea with the purity of 99.90% having a calcium content of 0.05 wt % obtained by recrystallization in advance was introduced, and the resulting solution was matured under reflux at 110 degree centigrade for 3 hours for producing a thiuronium salt. The solution was cooled down to 60 degree centigrade, and then 360.0 weight parts of toluene and 347.4 weight parts (5.10 mol) of 25 wt % aqueous ammonia solution were introduced thereinto for carrying out hydrolysis to obtain a toluene solution of polythiol having 4,8-dimercaptomethyl-1,11-dimercapto-3,6,9-trithiaundecane as a main ingredient. The toluene solution was subjected to acid washing and water washing for removing toluene and a trace of water under heat and reduced pressure. Thereafter, 198.8 weight parts of polythiol having 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane as main ingredients was obtained by filtering. APHA of the obtained polythiol was 10, while Y.I. thereof was 1.20.

Production of plastic lens

50.7 weight parts of m-xylylene diisocyanate, 0.01 weight part of dibutyltin dichloride as a curing catalyst, 0.10 weight part of Zelec UN (product name, acid phosphoric acid alkyl ester, a product of Stepan Co.) as an internal mold releasing agent and 0.05 weight parts of Viosorb 583 (product name, a product of Kyodo Chemical Co., Ltd.) as an ultraviolet absorber were mixed and dissolved at 20 degree centigrade. 49.3 weight parts of polythiol having 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane obtained as in the above as main ingredients was introduced thereinto, and the resulting solution was mixed to give a uniform mixed solution. This uniform solution was degassed at 600 Pa for 1 hour. Thereafter, the resulting solution was filtered using a 3-$\mu$m Teflon (registered trademark) filter, and then injected into a mold equipped with a glass mold and tapes. This mold was put into an oven and then gradually heated from 10 to 120 degree centigrade at which polymerization was conducted for 18 hours. After completion of polymerization, the mold was taken out from the oven and a resin was released from the mold. The obtained resin was additionally annealed at 120 degree centigrade for 3 hours. Y.I. of the obtained resin was 5.0 and the loss degree of transparency was 23. So, the evaluation was indicated with "o" on the loss degree of transparency.

Example 6

Polythiol having 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane as main ingredients was synthesized in the same manner as in Example 5, except that thiourea used in Example 2 was used instead of thiourea used in Example 5. APHA of the obtained polythiol was 10, while Y.I. thereof was 1.25. Using this polythiol, a plastic lens was produced and evaluated in the same manner as in Example 5. The evaluation results of the obtained plastic lens are shown in Table 1.

Example 7

Polythiol having 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane as main ingredients was synthesized in the same manner as in Example 5, except that thiourea used in Example 3 was used instead of thiourea used in Example 5. APHA of the obtained polythiol was 10, while Y.I. thereof was 1.33. Using this polythiol, a plastic lens was produced and evaluated in the same manner as in Example 4. The evaluation results of the obtained plastic lens are shown in Table 1.

Example 8

Polythiol having 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane as main ingredients was synthesized in the same manner as in Example 5, except that thiourea used in Example 4 was used instead of thiourea used in Example 5. APHA of the obtained polythiol was 10, while Y.I. thereof was 1.38. Using this polythiol, a plastic lens was produced and evaluated in the same manner as in Example 4. The evaluation results of the obtained plastic lens are shown in Table 1.

[Comparative Example 1]

Polythiol having 1,2-bis[(2-mercaptoethyl)thio]-3-mercaptopropane as a main ingredient was synthesized in the same manner as in Example 1, except that thiourea with the purity of 98.70% having a calcium content of 1.20 wt % was used instead of thiourea used in Example 1. APHA of the obtained polythiol was 20, while Y.I. thereof was 2.01. Using this polythiol, a plastic lens was produced and evaluated in the same manner as in Example 1. The evaluation results of the obtained plastic lens are shown in Table 1.

Comparative Example 2

Polythiol having 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane as main ingredients was synthesized in the same manner as in Example 5, except that thiourea with the purity of 98.70% having a calcium content of 1.20 wt % was used instead of thiourea used in Example 5. APHA of the obtained polythiol was 20, while Y.I. thereof was 2.10. Using this polythiol, a plastic lens was produced and evaluated in the same manner as in Example 5. The evaluation results of the obtained plastic lens are shown in Table 1.

[Table 1]

| Evaluation | Evaluation Results | | | | |
| --- | --- | --- | --- | --- | --- |
| | Calcium amount in thiourea (wt %) | APHA of polythiol | Y.I. of polythiol | Y.I. of plastic lens | Loss degree of transparency of plastic lens |
| Example 1 | 0.05 | 10 | 0.70 | 4.5 | 20 (o) |
| Example 2 | 0.20 | 10 | 0.81 | 4.7 | 22 (o) |
| Example 3 | 0.70 | 10 | 0.93 | 4.8 | 23 (o) |
| Example 4 | 0.90 | 10 | 0.95 | 5.0 | 26 (o) |
| Example 5 | 0.05 | 10 | 1.20 | 5.0 | 23 (o) |
| Example 6 | 0.20 | 10 | 1.25 | 5.3 | 26 (o) |
| Example 7 | 0.70 | 10 | 1.33 | 5.4 | 28 (o) |
| Example 8 | 0.90 | 10 | 1.38 | 5.5 | 29 (o) |
| Comparative Example 1 | 1.20 | 20 | 2.01 | 6.1 | 45 (×) |
| Comparative Example 2 | 1.20 | 20 | 2.10 | 6.8 | 50 (×) |

From the above results, the (poly)thiol compounds obtained by using thiourea having a calcium content of not more than 1.0 wt % were excellent in the color, and the plastic lenses produced by using this (poly)thiol compound were also excellent in the color and transparency. On the other hand, in the (poly)thiol compounds obtained by using thiourea having a calcium content in excess of 1 wt % in Comparative Examples 1 and 2, the color was worsened, while in the obtained plastic lenses, the color and transparency were worsened, either. The resins obtained in Examples and Comparative Examples were all colorless and transparent when respective resins were viewed, but resins of Comparative Examples were observed as slightly yellow in comparison with resins of Examples when all resins were compared.

INDUSTRIAL APPLICABILITY

According to the present invention, it is possible to produce a colorless and transparent (poly)thiol compound for an optical material in which coloring is suppressed, and a colorless and transparent (thio)urethane resin in which coloring and whitening are suppressed. The present invention greatly contributes to provision of optical materials and transparent materials, particularly plastic lenses for eyeglasses, in a stable manner.

**Claims**

1. A process for producing a (poly)thiol compound for an optical material comprising:

reacting an organic (poly)halogen compound or a (poly)alcohol compound with thiourea to produce an isothiuronium salt, and
hydrolyzing the obtained isothiuronium salt in the presence of aqeous ammonia to produce a (poly)thiol compound,

in which the calcium content in the thiourea is not more than 1.0 wt %.

2. The process for producing a (poly)thiol compound for an optical material as set forth in claim 1, in which said (poly)thiol compound has a sulfur atom in addition to a thiol group.

3. The process for producing a (poly)thiol compound for an optical material as set forth in claim 2, in which said (poly)thiol compound having a sulfur atom in addition to a thiol group has one or two or more kinds selected from the group consisting of 1,2-bis[(2-mercaptoethyl)thio]-3-mercaptopropane, 4,8-dimercaptomethyl-1,11-dimercapto-3,6,9-trithiaundecane, 4,7-dimercaptomethyl-1,11-dimercapto-3,6,9-trithiaundecane and 5,7-dimercaptomethyl-1,11-dimercapto-3,6,9-trithiaundecane as main ingredients.

4. The process for producing a (poly)thiol compound for an optical material as set forth in claim 1, 2 or 3, further comprising purifying thiourea to reduce the calcium content in the thiourea to 1.0 wt% or less, before said reacting step.

5. A process of producing a resin comprising a (poly)thiol compound by the process of any preceding claim, mixing it with a polyiso(thio)cyanate compound, and curing the resulting polymerizable composition to produce the resin.

6. The process of claim 5 used to produce an optical material comprising the resin.

7. The process of claim 5 used to produce a lens comprising the resin.

**Patentansprüche**

1. Verfahren zur Herstellung einer (Poly-)Thiolverbindung für ein optisches Material, umfassend:

das Umsetzen einer organischen (Poly-)Halogenverbindung oder einer (Poly-) Alkoholverbindung mit Thioharnstoff zur Herstellung eines Isothiuroniumsalzes und
das Hydrolysieren des erhaltenen Isothiuroniumsalzes in Gegenwart von wässrigem Ammoniak zur Herstellung einer (Poly-)Thiolverbindung,
wobei der Calciumgehalt im Thioharnstoff nicht mehr als 1,0 Gew.-% beträgt.

2. Verfahren zur Herstellung einer (Poly-)Thiolverbindung für ein optisches Material nach Anspruch 1, wobei die (Poly-)Thiolverbindung ein Schwefelatom zusätzlich zu der (den) Thiolgruppe(n) aufweist.

3. Verfahren zur Herstellung einer (Poly-)Thiolverbindung für ein optisches Material nach Anspruch 2, wobei die (Poly-)Thiolverbindung mit einem Schwefelatom zusätzlich zu der (den) Thiolgruppe(n) eine oder zwei weitere Verbindungen aus der aus 1,2-Bis[(2-mercaptoethyl)thio]-3-mercaptopropan, 4,8-Dimercaptomethyl-1,11-dimercapto-3,6,9-trithiaundecan, 4,7-Dimercaptomethyl-1,11-dimercapto-3,6,9-trithiaundecan, und 5,7-Dimercaptomethyl-1,11-dimercapto-3,6,9-trithiaundecan bestehenden Gruppe als Hauptbestandteil enthält.

4. Verfahren zur Herstellung einer (Poly-)Thiolverbindung für ein optisches Material nach einem der Ansprüche 1, 2 oder 3, das weiters das Reinigen des Thioharnstoffs zur Senkung des Calciumgehalts im Thioharnstoff auf 1,0 Gew.-% oder weniger vor dem Umsetzungsschritt umfasst.

5. Verfahren zur Herstellung eines Harzes, welches das Herstellen einer (Poly-) Thiolverbindung durch ein Verfahren nach einem der vorangegangenen Ansprüche, Vermischen derselben mit einer Polyiso(thio)cyanatverbindung und das Härten der resultierenden polymerisierbaren Zusammensetzung zur Herstellung des Harzes umfasst.

6. Verfahren nach Anspruch 5 zur Verwendung bei der Herstellung eines das Harz umfassenden optischen Materials.

7. Verfahren nach Anspruch 5 zur Verwendung bei der Herstellung einer das Harz umfassenden Linse.

**Revendications**

1. Procédé de production d'un composé de (poly)thiol pour un matériau optique, comprenant :

la mise en réaction d'un composé de poly(halogène) organique ou d'un composé de (poly)alcool avec de la thiourée pour produire un sel d'isothiuronium et

l'hydrolyse du sel d'isothiuronium obtenu en présence d'ammoniac aqueux pour produire un composé de (poly) thiol,

dans lequel la teneur en calcium de la thiourée est inférieure ou égale à 1,0 % en poids.

2. Procédé de production d'un composé de (poly)thiol pour un matériau optique selon la revendication 1, dans lequel ledit composé de (poly)thiol comprend un atome de soufre en plus d'un groupe thiol.

3. Procédé de production d'un composé de (poly)thiol pour un matériau optique selon la revendication 2, dans lequel ledit composé de (poly)thiol comprenant un atome de soufre en plus d'un groupe thiol comprend un ou deux types de composés ou plus choisis dans le groupe constitué par le 1,2-bis[(2-mercaptoéthyl)thio]-3-mercaptopropane, le 4,8-dimercaptométhyl-1,11-dimercapto-3,6,9-trithiaundécane, le 4,7-dimercaptométhyl-1,11-dimercapto-3,6,9-trithiaundécane et le 5,7-dimercaptométhyl-1,11-dimercapto-3,6,9-trithiaundécane en tant qu'ingrédients principaux.

4. Procédé de production d'un composé de (poly)thiol pour un matériau optique selon la revendication 1, 2 ou 3, comprenant également la purification de la thiourée pour réduire la teneur en calcium de la thiourée à 1,0 % en poids ou moins avant ladite étape de mise en réaction.

5. Procédé de production d'une résine comprenant la production d'un composé de (poly)thiol par le procédé selon l'une quelconque des revendications précédentes, le mélange de celui-ci avec un composé de polyiso(thio)cyanate, et le durcissement de la composition polymérisable résultante pour produire la résine.

6. Procédé selon la revendication 5, utilisé pour produire un matériau optique comprenant la résine.

7. Procédé selon la revendication 5, utilisé pour produire une lentille comprenant la résine.

**EP 2 008 998 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0378895 A
- JP 2001039944 A
- JP S48197349722 B
- JP H91997110955 B
- JP H91997110956 B
- JP H71995252207 B